**Europäisches Patentamt**

**European Patent Office**

**Office européen des brevets**

(11) Veröffentlichungsnummer: **0 053 313**
**B1**

(12) # EUROPÄISCHE PATENTSCHRIFT

(45) Veröffentlichungstag der Patentschrift:
25.09.85

(21) Anmeldenummer: **81109688.2**

(22) Anmeldetag: **14.11.81**

(51) Int. Cl.⁴: **A 61 K 9/16,** A 61 K 9/32,
A 61 K 31/71, A 23 K 1/17

(54) **Tierfutter enthaltend wachstumsfördernde Orthoester aus der Klasse der Orthosomycine in stabilisierter Form.**

(30) Priorität: **28.11.80 DE 3044800**

(43) Veröffentlichungstag der Anmeldung:
**09.06.82 Patentblatt 82/23**

(45) Bekanntmachung des Hinweises auf die Patenterteilung:
**25.09.85 Patentblatt 85/39**

(84) Benannte Vertragsstaaten:
**CH DE FR GB LI**

(56) Entgegenhaltungen:
**FR - A - 2 391 655**
**US - A - 3 892 849**

**R. VOIGT: "Lehrbuch der pharmazeutischen Technologie", 1973, VEB Verlag Volk und Gesundheit, Berlin, DD.**
**TETRAHEDRON, Band 35, 1979, Seiten 1207-1237, Pergamon Press Ltd., Oxford, G.B., D.E. WRIGHT: "The orthosomycins, a new family of antibiotics"**

(73) Patentinhaber: **BAYER AG, Konzernverwaltung RP Patentabteilung, D-5090 Leverkusen 1 Bayerwerk (DE)**

(72) Erfinder: **Voege, Herbert, Dr., Martin-Buber-Strasse 41, D-5090 Leverkusen 3 (DE)**
Erfinder: **Löffler, Hans Udo, Neustrasse 2a, D-5000 Köln 80 (DE)**
Erfinder: **Scheer, Martin, Dr., Herberts-Katernberg 7, D-5600 Wuppertal 1 (DE)**
Erfinder: **Gau, Wolfgang, Dr., Am Eckbusch 39/56, D-5600 Wuppertal 1 (DE)**

**Beschreibung**

Die Erfindung betrifft die Stabilisierung von Orthosomycinen in Tierfutter bzw. Tierfuttervormischungen durch Umhüllung mit einem magensaftunlöslichen Lack.

Für die Substanzklasse der Orthosomycine (siehe Tetrahedron Vo. 35, pp 1207–1237 D.F. Wright; DE-OS'en 2 510 161 und 2 510 160 sowie deutsche Patentanmeldung P 3 005 696.3) ist eine wachstumsfördernde Wirkung nachgewiesen. Ihr Einsatz war aber immer begrenzt durch die mangelhafte Stabilität der Wirkstoffe im Premix und besonders im Endfutter beim Verbraucher, wo der Wirkstoff über einen Zeitraum von 4 Monaten stabil sein soll. Während es beim Premix noch gelingen kann, die Stabilität negativ beeinflussender Substanzen wie Wasser fernzuhalten, ist dies beim Endfutter nicht möglich.

Es ist bekannt, Substanzen durch Umhüllen zu stabilisieren. So wurde z.B. Penicillin G durch Umhüllen mit Stearinsäure stabilisiert, um es Futter zumischen zu können. Viele Aromastoffe werden durch Mikroverkapseln mit Cellulose-Derivaten stabilisiert. Öllösliche Vitamine werden durch Einbetten in Polyglykole oder durch Mikroverkapseln mit Gelatine gegen Abbau stabilisiert.

Auch Acrylharze sind schon dazu benutzt worden, miteinander unverträgliche Substanzen dadurch kombinierbar zu machen, dass eine Substanz mit Acrylharz umhüllt wurde.

Es wurde nun gefunden, dass sich wachstumsfördernde Orthoester aus der Klasse der Orthosomycine durch Umhüllung mit einem magensaftunlöslichen Lack oder Film stabilisieren lassen. Überraschenderweise wird durch die Umhüllung mit einem magensaftunlöslichen Lack oder Film die Wirksamkeit der Substanz als Wachstumsförderer nicht beeinträchtigt.

Die Stabilisierung der Substanz im Endfutter und im Premix gerade durch diese Art Lack war nicht vorauszusehen. Versuche, den Wirkstoff z.B. mit wachsartigen Fettsäureestern oder Methylcellulose zu umhüllen, führten zu keiner Stabilisierung desselben.

Gegenstand der Erfindung ist demnach ein Tierfutter, enthaltend Orthoester aus der Klasse der Orthosomycine, die mit einem magensaftunlöslichen Lack oder Film umhüllt sind.

Als Wirkstoffe kommen alle Wachstumsförderer aus der Gruppe der Orthosomycine in Frage.

Dies sind insbesondere die in der eingangs genannten Literatur beschriebenen Wirkstoffe, ebenso wie solche, die durch H. Lächner und H. Wolf et al., Helvetica Chimica Acta – Vol. 62. Fasc. 1 (1979) – Nr. 1, S. 1ff, W.D. Ollis, et al., Helvetica Chmica Acta – Vol. 62, Fasc. 1 (1979) – Nr. 2 und H.L. Herzog et al., Applied Microbiology, July 1965, Vol. 13, Nr. 4, Seite 515ff, beschrieben werden.

Als Lacke sind prinzipiell alle solchen Polymeren geeignet, die im sauren Milieu des Magensaftes unlöslich sind, sich aber im mehr alkalischen Bereich auflösen oder zumindest stark verquellen. Filmbildner mit solchen Eigenschaften sind z.B. Schellack, Acrylharze, Methacrylate, Celluloseacetatphthalat, Polyvinylacetatphthalat, Mischpolymerisate aus Acrylnitril und Acrylsäure oder Mischpolymerisate aus Styrol und Maleinsäureanhydrid. Den filmbildenden Substanzen können übliche Weichmacher und andere Hilfsstoffe zugemischt werden. Als Weichmacher kommen z.B. Glycerin, Polyglykole, 1,2-Propylenglykol, Glycerintriacetat, Diethyl- und Dibutylphthalat u.a. in Frage. Es kann auch nützlich sein, der Lacksubstanz andere Hilfsmittel wie Talkum, Magnesiumstearat, Farbstoffe, kolloidale Kieselsäure, Antioxydantien, Zucker und Stärke zuzumischen.

Als Trägerstoffe für Vormischungen kommen die üblichen für Futtermittel eingesetzten Hilfsstoffe in Frage. So sind z.B. Getreidemehle, Getreidenachmehle, gemahlene Schalen aus Sojabohnen oder Mandeln, aber auch anorganische Trägerstoffe wie sek.-Calciumphosphat, Natriumbicarbonat oder Calciumcarbonat geeignet.

Die Verfahren zur Herstellung von Premixen aus diesen mit einer Schutzschicht umhüllten Orthosomycinen sind bekannt. So können die Wirkstoffe zusammen mit der Hüllsubstanz z.B. sprühgetrocknet werden. Nach einem anderen Verfahren wird auf ein Fliessbett aus Wirkstoff oder aus Wirkstoff mit Hilfsstoffen die umhüllende Substanz aufgesprüht und dabei getrocknet.

Der Anteil des Wirkstoffes im Premix kann beliebig variieren zwischen 0,5 Gew.-% und 90 Gew.-%, vorzugsweise jedoch zwischen 1 und 30 Gew.-%.

Der Anteil Filmbildner oder Lackbildner kann über einen grossen Bereich variieren, da je nach Herstellungsverfahren unterschiedliche Mengen nötig sind. So kann der Lack oder Film, bezogen auf das zu umhüllende Material, zwischen 0,1 Gew.-% und 30 Gew.-% liegen. Bevorzugt wird ein Bereich von 0,3 Gew.-% bis 10 Gew.-%.

Die anderen Hilfsstoffe, z.B. der Weichmacher für den Lack oder Film werden in Mengen kleiner als 3 Gew.-% eingesetzt – vorzugsweise kleiner als 1 Gew.-%.

Beispiel 1

5,0 Gew.-% Wirkstoff werden mit 84,8 Gew.-% Natriumbicarbonat gemischt und mit einer Acrylharzlösung im Wirbelschichtgranulator besprüht bis ein Acrylharzanteil von 10,2 Gew.-% aufgetragen ist. Die Zulufttemperatur beträgt dabei 90 °C, die Produkttemperatur 30–35 °C. Anschliessend wird noch getrocknet bis die Produkttemperatur auf 50 °C gestiegen ist. Dann wird abgekühlt.

Es hat sich ein homogenes, feinkörniges Premix gebildet.

Beispiel 2

5 Gew.-% Wirkstoff werden in einer Acrylharz-Dispersion homogenisiert und unter den gleichen Bedingungen wie bei Beispiel 1 auf Weizennachmehl als Trägerstoff aufgesprüht.

Beispiel 3

Es werden 30 Gew.-% Wirkstoff mit 3 Gew.-% Lack und 0,03 Gew.-% Triacetin als Weichmacher in Wasser homogenisiert und über einen Sprühtrockner versprüht. Das feinkörnige Produkt wird mit Weizennachmehl zu einem 10%igen Premix abgemischt.

Beispiel 4

Vergleich der Stabilität im Endfutter von reinem Wirkstoff zu Wirkstoff, der nach Beispiel 1 und Beispiel 2 umhüllt wurde.

| | Anfangs-wert | 10 Wochen bei 20°C | 35°C | 6 Monate bei 20°C | 35°C |
|---|---|---|---|---|---|
| reiner Wirkstoff in Ferkelaufzuchtfutter | 38 ppm | 11 ppm | 6,5 ppm | 3,4 ppm | nicht nach-weisbar |
| umhüllter Wirkstoff nach Beispiel 1 in Ferkel-aufzuchtfutter | 36 ppm | 33 ppm | 22 ppm | 31 ppm | 11 ppm |
| umhüllter Wirkstoff nach Beispiel 2 in Ferkel-aufzuchtfutter | 55 ppm | 55 ppm | 55 ppm | 48 ppm | 46 ppm |

**Patentansprüche**

1. Tierfutter enthaltend Orthoester aus der Klasse der Orthosomycine umhüllt mit einem magensaftunlöslichen Lack oder Film.

2. Tierfutter gemäss Anspruch 1, dadurch gekennzeichnet, dass der Anteil des Lacks oder Films 0,1 bis 30 Gew.-% bezogen auf die zu umhüllenden Orthosomycine beträgt.

3. Tierfutter gemäss Anspruch 1, dadurch gekennzeichnet, dass der Anteil des Lacks oder Films 0,3 bis 10 Gew.-%, bezogen auf die zu umhüllenden Orthosomycine, beträgt.

4. Tierfutter gemäss Anspruch 1, dadurch gekennzeichnet, dass der Lack oder Film aus einem magensaftunlöslichen Acrylharz besteht.

5. Tierfutter gemäss Anspruch 1, dadurch gekennzeichnet, dass der Lack oder Film aus Schellack, Methacrylat, Celluloseacetatphthalat, Polyvinylacetatphthalat, Acrylnitril-Acrylsäure, Mischpolymerisat oder Styrol-Maleinsäureanhydrid-Mischpolymerisat besteht.

6. Tierfutter gemäss Anspruch 5, dadurch gekennzeichnet, dass der Lack oder Film Weichmacher und Hilfsmittel wie Talkum, Magnesiumstearat, Farbstoffe, kolloidale Kieselsäure, Antioxydantien, Zucker und/oder Stärke enthält.

7. Tierfuttervormischung bestehend aus Getreidemehlen, Getreidenachmehlen oder gemahlenen Schalen aus Sojabohnen oder Mandeln, enthaltend Orthoester aus der Klasse der Orthosomycine, umhüllt mit einem magensaftunlöslichen Lack oder Film.

**Claims**

1. Animal feed containing ortho esters from the class of orthosomycins coated with a lacquer or film which is insoluble in gastric juice.

2. Animal feed according to Claim 1, characterised in that the proportion of the lacquer or film is 0.1 ot 30% by weight, relative to the orthosomycins to be coated.

3. Animal feed according to Claim 1, characterised in that the proportion of the lacquer or film is 0.3 to 10% by weight, relative to the orthosomycins to be coated.

4. Animal feed according to Claim 1, characterised in that the lacquer or film consists of an acrylic resin which is insoluble in gastric juice.

5. Animal feed according to Claim 1, characterised in that the lacquer or film consists of shellac, methacrylate, cellulose acetate phthalate, polyvinyl acetate phthalate, acrylonitrile-acrylic acid copolymer or styrene-maleic anhydride copolymer.

6. Animal feed according to Claim 5, characterised in that the lacquer or film contains plasticisers and auxiliaries such as talc, magnesium stearate, colorants, colloidal silicic acid, antioxidants, sugar and/or starch.

7. Animnal feed premix consisting of cereal flours, cereal middlings or ground shells of soyabeans or almonds, containing ortho esters from the class of orthosomycins coated with a lacquer or film which is insoluble in gastric juice.

**Revendications**

1. Aliment pour le bétail contenant un orthoester de la classe des orthosomycines, enrobé d'un revêtement ou d'un film insoluble dans le suc gastrique.

2. Aliment pour le bétail suivant la revendication 1, caractérisé en ce que la proportion de revêtement ou de film est de 0,1 à 30% en poids par rapport aux orthosomycines à envelopper.

3. Aliment pour le bétail suivant la revendication 1, caractérisé en ce que la proportion de revêtement ou de film s'élève à 0,3–10% en poids par rapport aux orthosomycines à envelopper.

4. Aliment pour le bétail suivant la revendication 1, caractérisé en ce que le revêtement ou le film est formé d'une résine acrylique insoluble dans le suc gastrique.

5. Aliment pour le bétail suivant la revendication 1, caractérisé en ce que le revêtement ou le film est formé de gomme laque raffinée, de méthacrylate, d'acétophtalate de cellulose, d'acétophtalate de polyvinyle, d'un copolymère d'acrylonitrile et d'acide acrylique ou d'un copolymère de styrène et d'anhydride d'acide maléique.

6. Aliment pour le bétail suivant la revendication 5, caractérisé en ce que le revêtement ou le film contient un plastifiant et des adjuvants gels que du talc, du stéarate de magnésium, des colorants, de la silice colloïdale, des anti-oxydants, du sucre et/ou de l'amidon.

7. Mélange préalable pour aliment pour le bétail, composé de farines de cèréales, de farines de céréales de qualité inférieure ou de coques broyées de soja ou d'amandes, contenant des ortho-esters de la classe des orthosomycines, enveloppés d'un revêtement ou d'un film insoluble dans le suc gastrique.